# EUROPÄISCHE PATENTANMELDUNG

(11) **EP 1 719 475 A1**
(43) Veröffentlichungstag der Anmeldung: **08.11.2006**
(21) Anmeldenummer: 05009817.7
(22) Anmeldetag: 04.05.2005
(51) Int. Cl.: A61F 2/14, A61F 2/16

(54) **Augenimplantat**

(71) Anmelder: Carriazo, Cesar C., Dr., Baranquilla (CO)
(72) Erfinder: Carriazo, Cesar C., Dr., Baranquilla (CO)
(74) Vertreter: Hofstetter, Alfons J.

(57) **Zusammenfassung**

Die Erfindung betrifft ein künstliches Augenimplantat, insbesondere als Ersatz einer Augenlinse oder eines Glaskörpers eines menschlichen oder tierischen Auges (16) bestehend aus einem Hohlkörper (12), wobei der Hohlkörper (12) aus einem bioverträglichen, flexiblen und dehnbaren Material besteht und mit einem Medium (14) befüllbar ist.

## Beschreibung

Die vorliegende Erfindung betrifft ein künstliches Augenimplantat, insbesondere als Ersatz einer Augenlinse oder eines Glaskörpers eines menschlichen oder tierischen Auges bestehend aus einem Hohlkörper.

Die als Katarakt (grauer Star) bezeichnete Eintrübung der Augenlinse ist heute eine sehr häufige Erkrankung, von der insbesondere ältere Menschen betroffen sind. Die einzige Möglichkeit der Korrektur ist die chirurgische Behandlung. Sie besteht in der Entfernung von vorderer Linsenkapsel, Linsenrinde und Linsenkern mit anschließender Implantation einer sog. lntraokularlinse (IOL), die in den Linsenkapselsack eingeführt wird und aus unterschiedlichen Materialien bestehen kann. Was die Wiederherstellung der Sehschärfe angeht, so werden mit dieser Methode sehr gute Erfolge erzielt. Was jedoch die Wiederherstellung der normalen Akkomodationsfunktion der Linse mit der Möglichkeit einer Fokussierung im Nah- und Fernbereich ohne die Notwendigkeit korrigierender Sehhilfen betrifft, so sind die Ergebnisse chirurgischer Eingriffe noch immer ungenügend.

In der chirurgischen Kataraktbehandlung besteht heute weltweit die Tendenz, den Eingriff unter örtlicher Betäubung und mit immer kleineren Schnitten durchzuführen. Auf diese Weise kann auf das Anbringen von Nähten verzichtet werden, die die Wiederherstellung der Sehkraft verzögern und zur Ausbildung einer Stabsichtigkeit (Astigmatismus) führen können. Gleichzeitig wurde eine Vielzahl von IOL-Typen entwickelt, die eine Wiederherstellung der Akkomodationsfähigkeit ermöglichen sollten. Hierzu zählen verschiedene Linsendesigns mit kleinen elastischen Haltebeinen (sog. Haptiken) unterschiedlicher Form, die eine mittige Ausrichtung innerhalb des Kapselsacks ermöglichen sollen (z. B. DE 35 03 690 A1, DE 36 35 111 A1, US 4725277 A, US 4842602 A). Der Einsatz dieser Linsenkonstruktionen erbrachte jedoch keine zufrieden stellenden Ergebnisse, da die Ausbildung einer Kapselsackfibrose die Positionsänderung der Intraokularlinse (IOL) einschränkt.

Auch die Entwicklung verschiedener IOL mit einer optischen Zone für den Nah- und Fernbereich sowie für den mittleren Sehbereich erwies sich als nur bedingt erfolgreich, da die betreffenden IOL (z.B. DE 40 38 088 A1, US 5628798 A, US 2003/0149480 A1, US 2004/0148022 A1) nicht im eigentlichen Sinne akkomodationsfähig sind.

Seit einigen Jahren denkt man auch daran, Kunstlinsen aus dehnungsfähigen Materialien herzustellen. Vor diesem Hintergrund experimentierte man mit verschiedenen Linsentypen aus flexiblen Materialien, mit elastischen Filtern und der Möglichkeit, die Brechkraft im Anschluss an die Implantation durch Lichtbestrahlung patientengerecht einzustellen und aus Materialien, die sich temperaturabhängig ausdehnen (Smart-Linse, z.B. US 2005/021139 A1). Diese Linsenkonstruktionen, die ein Einführen des Implantats über sehr kleine Schnitte ermöglichen sollen, werden derzeit aber noch auf ihre Bioverträglichkeit mit menschlichem Gewebe und auf ihr Verhalten im Auge getestet.

Bei dem Versuch, die menschliche Augenlinse durch Kunstlinsen von vergleichbarer Größe zu ersetzen, besteht der limitierende Faktor bislang in dem Umstand, dass hierfür große operative Einschnitte erforderlich sind, während weltweit die gegenläufige Tendenz zur Durchführung sehr kleiner Schnitte zu beobachten ist. Auch die Methode einer Flüssigkeitsinjektion direkt in den Kapselsack stößt hauptsächlich aus zwei Gründen an ihre Grenzen. Zum einen darf der Kapselsack während des Einspritzens der Flüssigkeit nicht geöffnet sein. Genau dies aber ist stets der Fall, da für die Kataraktextraktion die Durchführung einer vorderen Kapsulorhexis (Eröffnung der vorderen Linsenkapsel) erforderlich ist, so dass die Flüssigkeit in die vordere Augenkammer austreten würde. Zum anderen müssen sich die eingespritzten Flüssigkeiten innerhalb des Auges verfestigen, um Linsenfunktion übernehmen zu können. Hierfür sind jedoch eine Reihe chemischer Reaktionen erforderlich, die im Augeninneren nicht ablaufen können.

Zu den heute am häufigsten verwendeten Flüssigkeiten, mit denen nach einer Netzhautablösung die Netzhaut wieder zum Anliegen gebracht wird, gehört Silikonöl, das ähnliche Eigenschaften wie der Glaskörper besitzt. Da Silikonöl eine höhere Dichte hat als Wasser, wird es bei Patienten mit Netzhautablösung nach der operativen Entfernung des Glaskörpers (Vitrektomie) als Glaskörperersatz verwendet, um so die Netzhaut wieder zum Anliegen zu bringen. Silikonöl wird zwar heute häufig eingesetzt, hat aber dennoch zwei große Nachteile. Der erste Nachteil resultiert aus dem Umstand, dass im Falle bestehender Netzhautperforationen das Silikonöl trotz seiner hohen Dichte hinter die Netzhaut eindringen und auf diese Weise eine erneute Netzhautablösung hervorrufen kann. Der zweite Nachteil besteht in der Tatsache, dass es nach mehrmonatigem Kontakt mit den im Augeninneren vorhandenen Flüssigkeiten zu einem Emulgierungsprozess und zur Retinatoxizität kommt, so dass es erforderlich wird, das Silikonöl aus dem Auge zu entfernen.

Aufgabe der Erfindung ist es daher ein Augenimplantat der eingangs genannten Art bereitzustellen, welches die genannten Nachteile herkömmlicher Augenimplantate überwindet und insbesondere einen akkomodationsfähigen Ersatz einer Augenlinse oder eines Glaskörpers eines menschlichen oder tierischen Auges bereitstellt, wobei der Einsatz des Implantats minimalinvasiv durchgeführt werden kann.

Gelöst wird diese Aufgabe durch ein gattungsgemäßes künstliches Augenimplantat mit den Merkmalen des Anspruchs 1.

Vorteilhafte Ausgestaltungen des künstlichen Augenimplantats sind in den Unteransprüchen beschrieben.

Ein erfindungsgemäßes künstliches Augenimplantat, insbesondere als Ersatz einer Augenlinse oder eines Glaskörpers eines menschlichen oder tierischen Auges, besteht aus einem Hohlkörper, wobei der Hohlkörper aus einem bioverträglichen, flexiblen und dehnbaren Material besteht und mit einem Medium befüllbar ist. Durch eine derartige Ausgestaltung des Augenimplantats ist gewährleistet, dass es in nicht befülltem Zustand sehr leicht innerhalb des Auges angebracht werden kann, so dass ein entsprechender Eingriff am menschlichen oder tierischen Auge minimalinvasiv durchgeführt werden kann. Des Weiteren ist durch das erfindungsgemäße Augenimplantat gewährleistet, dass eine Wiederherstellung der Akkomodationsfähigkeit des Patienten erfolgt. Durch die Einführung des Hohlkörpers als Ersatz der Augenlinse und durch die Ausfüllung des gesamten Kapselsacks lässt sich die Vibrosierung von Vorderkapsel und Hinterkapsel vermeiden, so dass der Ziliarmuskel des Auges seine Akkomodationsaufgabe wieder übernehmen kann. Da er der Verwendung des künstlichen Augenimplantats als Ersatz einer Augenlinse dient, ist der Hohlkörper in dem mit einem Medium gefüllten Zustand linsenförmig ausgebildet und weist insgesamt im Hinblick auf Dicke, Biegsamkeit, Brechkraft und Akkomodationsvermögen ähnliche Eigenschaften auf wie die natürliche menschliche oder tierische Augenlinse. Zudem sind für das erfindungsgemäße Augenimplantat keine Befestigungsvorrichtungen wie z.B. Haptiken notwendig, da das Augenimplantat die Position der natürlichen Augenlinse voll einnimmt und damit innerhalb des Kapselsacks automatisch fixiert ist. Wird das erfindungsgemäße künstliche Augenimplantat als Ersatz eines Glaskörpers des menschlichen oder tierischen Auges verwendet, so bildet der Hohlkörper in einem mit dem Medium gefüllten Zustand ungefähr die Form des Glaskörpers des menschlichen oder tierischen Auges nach. So wird das erfindungsgemäße Augenimplantat nach dem Einbringen in die Augenhöhle mit Silikonöl oder mit einer anderen Flüssigkeit gefüllt. Durch das als Hohlkörper ausgebildete Augenimplantat ist zudem gewährleistet, dass die eingebrachte Flüssigkeit zum einen nicht in Kontakt mit den im Augeninneren vorhandenen Gewebestrukturen und Flüssigkeiten gerät und zum anderen nicht hinter die Netzhaut fließen kann, da sie vollständig in dem Hohlraum eingeschlossen ist. Aus diesem Grund ist es auch vorteilhafterweise nicht notwendig, sie später wieder aus dem Auge zu entfernen.

In einer vorteilhaften Ausgestaltung der Erfindung weist der Hohlkörper mindestens eine Ventilvorrichtung zum Befüllen des Hohlraums mit dem Medium auf. Dadurch ist es ohne Komplikationen möglich, den Hohlkörper mit dem entsprechenden Medium zu befüllen, ohne dass das Medium in das umliegende Augengewebe gelangt. Die Befüllung kann zum Beispiel durch eine mit dem Medium gefüllte Spritze und eine entsprechende Kanüle erfolgen.

In einer weiteren vorteilhaften Ausgestaltung weist der Hohlkörper mindestens eine Schlauchverbindung zum Einfüllen des Mediums in den Hohlraum auf. Die Schlauchverbindung kann zum Beispiel als flexibler Mikroschlauch ausgebildet sein. So kann vorteilhafterweise nach dem Einbringen des Augenimplantats in den Kapselsack des Auges der Hohlraum über den Mikroschlauch kontrolliert mit dem Medium befüllt werden. Nach Abschluss des Füllvorgangs kann der Schlauch wieder entfernt werden.

Es ist aber auch möglich, dass der Hohlkörper des erfindungsgemäßen künstlichen Augenimplantats weder eine Ventilvorrichtung noch eine Schlauchverbindung zum Befüllen des Hohlraums mit dem Medium aufweist. So kann das bioverträgliche Material des Hohlkörpers derart gewählt werden, dass es sich aufgrund seiner elastischen Eigenschaften von selbst schließt, wenn zum Beispiel eine Injektionsnadel nach dem Füllvorgang entfernt wird. Die oben genannte Schlauchverbindung kann unter anderem auch über die ebenfalls genannte Ventilvorrichtung angeschlossen sein, so dass sich der Hohlkörper nach dem Entfernen der Schlauchverbindung ebenfalls wieder von selbst schließt.

In einer weiteren vorteilhaften Ausgestaltung der Erfindung weist der Hohlkörper an seiner dem Augeninneren abgewandten Seite mindestens eine Vorrichtung mit optischer Brechkraft auf. Des Weiteren ist es möglich, dass der Hohlkörper auch oder ausschließlich an seiner dem Augeninneren zugewandten Seite mindestens eine Vorrichtung mit optischer Brechkraft aufweist. Durch die entsprechende Ausgestaltung oder Modellierung der Vorder- und/oder der Rückseite des Hohlkörpers bzw. Augenimplantats können optische Korrekturen gezielt in Abhängigkeit von den Patientenbedürfnissen durchgeführt werden. Auch der Brechungsindex des Mediums kann den Patientenbedürfnissen angepasst werden.

In einer vorteilhaften Ausgestaltung des erfindungsgemäßen künstlichen Augenimplantats ist das Medium flüssig und/oder halb-flüssig und/oder gasförmig. Beispielhaft seien hier physiologische Flüssigkeiten, Silikongele und -öle, Polymerverbindungen und Edelgase genannt. Grundsätzlich kann als Medium jedes Material verwendet werden, das einerseits sich zur Füllung des Hohlkörpers eignet und andererseits vordefinierte physikalische Eigenschaften, wie zum Beispiel den genannten Brechungsindex, aufweisen. Lediglich Medien, die das Material des Hohlkörpers selbst angreifen bzw. damit chemisch reagieren, können nicht als Medium verwendet werden.

Weitere Vorteile, Einzelheiten und Merkmale des erfindungsgemäßen Augenimplantats werden anhand mehrerer in den Figuren dargestellter Ausführungsformen beispielhaft dargestellt. Es zeigen
- Figur 1: eine schematische Darstellung eines menschlichen Auges;
- Figur 2: eine schematische Darstellung des menschlichen Auges nach Entfernung der Augenlinse;
- Figur 3: eine schematische Darstellung eines menschlichen Auges mit einem implantierten künstlichem Augenimplantat gemäß dem Stand der Technik;
- Figur 4: eine schematische Darstellung eines erfindungsgemäßen Augenimplantats gemäß einer ersten Ausführungsform während dem Einführen in das menschliche Auge;
- Figur 5: eine schematische Darstellung des erfindungsgemäßen Augenimplantats gemäß Figur 4 beim Einführen in die Linsenkapsel des Auges;
- Figur 6: eine schematische Darstellung des erfindungsgemäßen Augenimplantats gemäß Figur 4 mit angeschlossener Befüllvorrichtung;
- Figur 7: eine schematische Darstellung des erfindungsgemäßen Augenimplantats gemäß Figur 4 in einem mediumbefüllten Zustand;
- Figur 8: eine schematische Darstellung eines erfindungsgemäßen Augenimplantats gemäß einer zweiten Ausführungsform in einem mediumbefüllten Zustand;
- Figur 9: eine schematische Darstellung eines erfindungsgemäßen Augenimplantats gemäß einer dritten Ausführungsform während dem Einführen in eine hintere Augenhöhle des menschlichen Auges;
- Figur 10: eine schematische Darstellung des erfindungsgemäßen Augenimplantats gemäß Figur 9 mit angeschlossener Befüllvorrichtung;

- Figur 11: eine schematische Darstellung des erfindungsgemäßen Augenimplantats gemäß Figur 9 in einem mediumbefüllten Zustand; und
- Figur 12: eine Darstellung eines erfindungsgemäßen Augenimplantats gemäß einer vierten Ausführungsform.

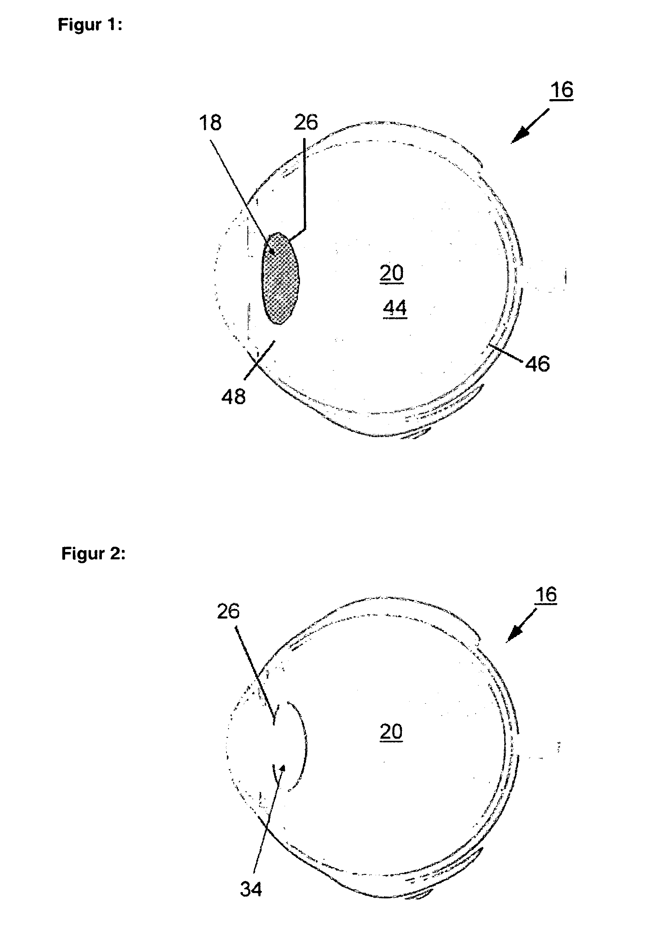

Figur 1 zeigt eine schematische Darstellung eines menschlichen Auges 16. Man erkennt die Lage der Augenlinse 18 mit den zur Akkomodation der Linse 18 notwendigen Ziliarmuskeln 48. Die Augenlinse 18 ist dabei in einer Linsenkapsel bzw. einem Kapselsack 26 gelagert. Des Weiteren umfasst das Augeninnere einen Glaskörper 20 innerhalb des Glaskörperraums 24 sowie die den Glaskörperraum 24 umgebende Netzhaut 46.

Figur 2 zeigt eine schematische Darstellung des menschlichen Auges 16 nach Entfernung der Augenlinse 18. Man erkennt, dass der Kapselsack 26 nunmehr geöffnet ist und innerhalb des Kapselsacks 26 sich ein Hohlraum 34 ausbildet. Die Entfernung der Augenlinse 18 kann zum Beispiel durch ihre Eintrübung und damit den Verlust der Funktionsfähigkeit notwendig sein.

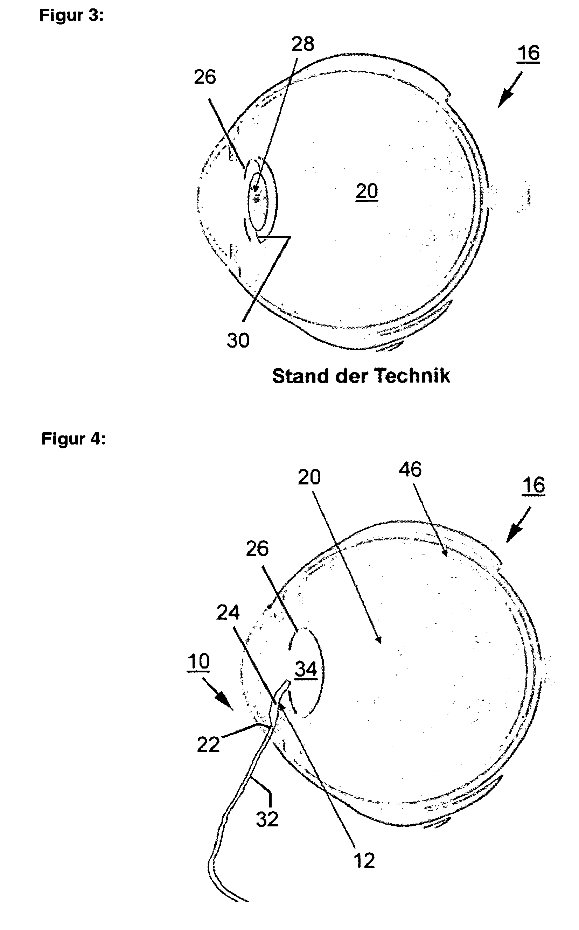

Figur 3 zeigt eine schematische Darstellung eines menschlichen Auges 16 mit einem implantierten künstlichen Augenimplantat 28 gemäß dem Stand der Technik. Man erkennt, dass das künstliche Augenimplantat mehrere Haptiken 30 zur Lagefixierung innerhalb des Kapselsacks 26 aufweist. Die Nachteile derartiger bekannter Augenimplantate wurden bereits im Vorhergehenden beschrieben.

Figur 4 zeigt eine schematische Darstellung eines künstlichen Augenimplantats 10 gemäß einer ersten Ausführungsform der Erfindung. Die Darstellung zeigt dabei das Augenimplantat 10 während dem Einführen in das menschliche Auge 16. Das Einführen erfolgt dabei über einen kleinen Schnitt, der bereits zur Entfernung der Augenlinse 18 benutzt wurde. Man erkennt, dass das in dem dargestellten Ausführungsbeispiel gezeigte Augenimplantat 10 als Ersatz der Augenlinse dient und einen Hohlkörper 12 umfasst. Der Hohlkörper 12 besteht dabei aus einem bioverträglichen, flexiblen und dehnbaren Material und ist mit einem Medium 14 befüllbar. Als Materialien für das künstliche Augenimplantat kommen insbesondere bioverträgliche, flexible und dehnbare Kunststoffe, Silikone und eine Vielzahl anderer Polymerverbindungen infrage, die die geforderten Eigenschaften aufweisen. Beispielhaft seien hier Silikongummi, Acrylat, Polyurethan, Latex und Polymethylmetaacrylat (PMMA) genannt.

Des Weiteren erkennt man, dass das Augenimplantat 10 eine Ventilvorrichtung 22 zum Befüllen des Hohlraums 24 mit dem Medium 14 aufweist. Mit der Ventilvorrichtung 22 lösbar verbunden ist eine Schlauchverbindung 32, die wiederum zu einer Mediumsquelle führt.

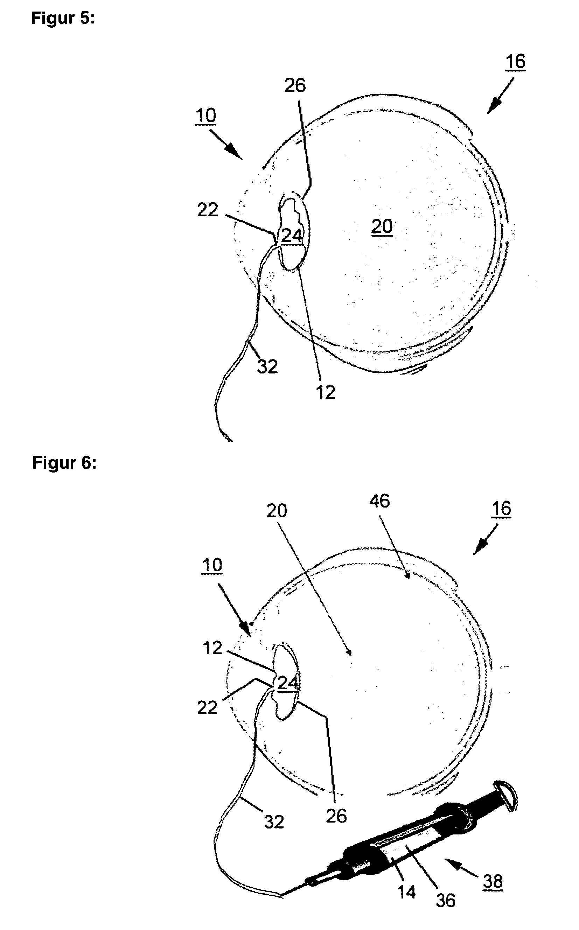

Figur 5 zeigt eine schematische Darstellung des Augenimplantats 10 gemäß Figur 4 beim Einführen in die Linsenkapsel 26 bzw. den Hohlraum 34 der Linsenkapsel 26. Man erkennt, dass der Hohlkörper 12 mit seinem Hohlraum 24 sich innerhalb des Kapselsacks 26 ausdehnt.

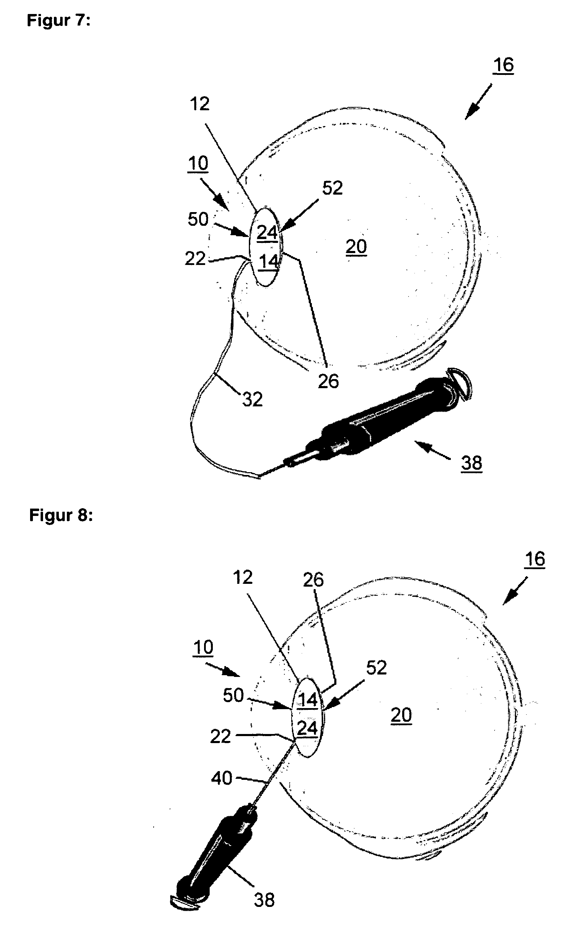

In Figur 6 wird das Augenimplantat 10 gemä0 Figur 4 mit angeschlossener Befüllvorrichtung 38 gezeigt. Man erkennt, dass die Befüllvorrichtung 38 einen Vorratsraum 36 mit dem Medium 14 aufweist. Über eine entsprechende Verbindung der Befüllvorrichtung 38 mit der Schlauchverbindung 32 erfolgt dann ein Befüllen des Hohlkörpers 12 des Augenimplantats 10 (vergleiche Figur 7). Nach dem vollständigen Befüllen des Hohlkörpers 12 bzw. des Hohlraums 24 des Hohlkörpers 12 mit dem Medium 14 bildet der Hohlkörper 12 ungefähr die Form der vorher entfernten Augenlinse nach. Das Augenimplantat weist im gefüllten Zustand eine gekrümmte Vorderwand 50 und eine dem Glaskörper 20 zugewandte Rückwand 52 auf. Nach der vollständigen Befüllung des Augenimplantats 10 wird die Schlauchverbindung 32 von dem Hohlkörper 12 gelöst. Die Ventilvorrichtung 22 sorgt dafür, dass es zu keinem Austritt des Mediums 14 in das umgebende Augengewebe kommt. Das Medium 14 kann in dem dargestellten Ausführungsbeispiel, wie auch bei den noch folgenden Ausführungsbeispielen, flüssig und/oder halb-flüssig und/oder gasförmig sein. Auch andere Aggregatszustände des Mediums sind denkbar.

Figur 8 zeigt eine schematische Darstellung des Augenimplantats 10 gemäß einer zweiten Ausführungsform in einem mediumbefüllten Zustand. Man erkennt, dass anstelle der Schlauchverbindung 32 das Medium 14 nunmehr mittels einer Kanüle 40 einer spritzenartigen Befüllvorrichtung 38 befüllt wird. Dabei dringt die Kanüle 40 in die Ventilvorrichtung 22 des Hohlkörpers 12 ein und bringt so das Medium 14 in den Hohlraum 24 des Hohlkörpers 12. Nach vollständiger Befüllung des Hohlraums 24 wird die Befüllvorrichtung 38 zurückgezogen und die Ventilvorrichtung 22 verschließt den Hohlkörper 12. Man erkennt, dass das Augenimplantat 10 im vollständig befüllten Zustand wiederum eine gekrümmte Vorderwand 50 und eine dem Glaskörper 20 zugewandte gekrümmte Rückwand 52 aufweist.

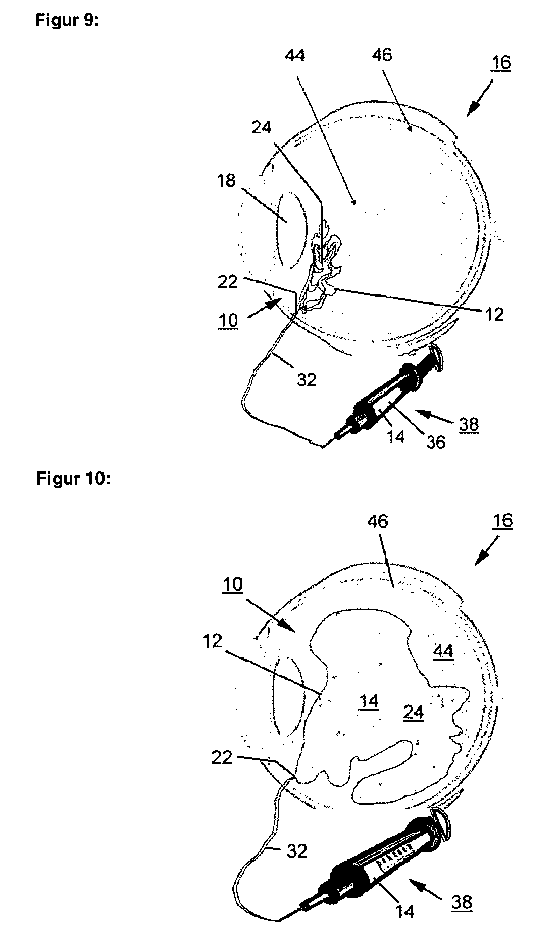

Figur 9 zeigt eine schematische Darstellung eines Augenimplantats 10 gemäß einer dritten Ausführungsform während dem Einführen in eine hintere Augenhöhle 44 des menschlichen Auges 16. Aus der hinteren Augenhöhle 44 wurde der Glaskörper 20 entfernt. Über einen kleinen Schnitt, über den bereits der Glaskörper 20 entfernt wurde, wird das Augenimplantat 10 bestehend aus dem Hohlkörper 12 mit dem Hohlraum 24 in die hintere Augenhöhle 44 eingeführt. Über die Ventilvorrichtung 22, die daran lösbar angeschlossene Schlauchverbindung 32 und die mit der Schlauchverbindung 32 verbundene Befüllvorrichtung 38 wird anschließend das Medium 14 in den Hohlraum 24 des Hohlkörpers 12 eingefüllt (vergleiche Figur 10).

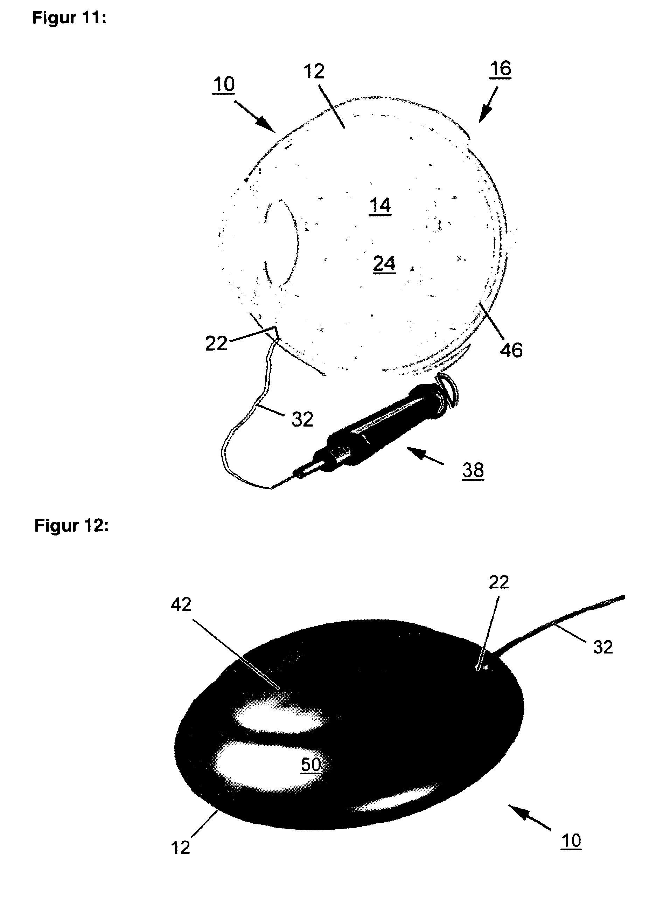

In einem in Figur 11 dargestellten, völlig mit dem Medium 14 befüllten Zustand bildet der Hohlkörper 12 ungefähr die Form des Glaskörpers 20 des menschlichen oder tierischen Auges 16 nach und liegt an der Netzhaut 46 an. Nach dem vollständigen Befüllen des Hohlkörpers 12 wird die Schlauchverbindung 32 von dem Augenimplantat 10 bzw. dessen Ventilvorrichtung 22 gelöst.

Figur 12 zeigt eine Darstellung eines Augenimplantats 10 gemäß einer vierten Ausführungsform. Man erkennt, dass der Hohlkörpers 12 an seiner dem Augeninneren abgewandten Seite 50 eine Vorrichtung 42 mit optischer Brechkraft aufweist. In dem dargestellten Ausführungsbeispiel handelt es sich um eine optische Linse, die einstückig mit dem Hohlkörper 12 ausgebildet ist. Des Weiteren erkennt man die Schlauchverbindung 32, die lösbar an der Ventilvorrichtung 22 mit dem Hohlkörper 32 verbunden ist. In dem dargestellten Ausführungsbeispiel dient das Augenimplantat 10 als Ersatz einer Augenlinse.

## Patentansprüche

1. Künstliches Augenimplantat, insbesondere als Ersatz einer Augenlinse oder eines Glaskörpers eines menschlichen oder tierischen Auges bestehend aus einem Hohlkörper (12),
**dadurch gekennzeichnet,**
**dass** der Hohlkörper (12) aus einem bioverträglichen, flexiblen und dehnbaren Material besteht und mit einem Medium (14) befüllbar ist.

2. Augenimplantat nach Anspruch 1,
**dadurch gekennzeichnet,**
**dass** der Hohlkörper (12) in einem mit dem Medium (14) gefüllten Zustand linsenförmig ausgebildet ist.

3. Augenimplantat nach Anspruch 1,
**dadurch gekennzeichnet,**
**dass** der Hohlkörper (12) in einem mit dem Medium (14) gefüllten Zustand ungefähr die Form des Glaskörpers (20) des menschlichen oder tierischen Auges (16) nachbildet.

4. Augenimplantat nach Anspruch einem der vorhergehenden Ansprüche,
**dadurch gekennzeichnet,**
**dass** der Hohlkörper (12) mindestens eine Ventilvorrichtung (22) zum Befüllen des Hohlraums (24) mit dem Medium (14) aufweist.

5. Augenimplantat nach einem der vorhergehenden Ansprüche,
**dadurch gekennzeichnet,**
**dass** der Hohlkörper (12) mindestens eine Schlauchverbindung (32) zum Einfüllen des Mediums (14) in den Hohlraum (24) aufweist.

6. Augenimplantat nach einem der vorhergehenden Ansprüche,
**dadurch gekennzeichnet,**
**dass** der Hohlkörper (12) an seiner dem Augeninneren abgewandten Seite mindestens eine Vorrichtung (42) mit optischer Brechkraft aufweist.

7. Augenimplantat nach einem der vorhergehenden Ansprüche,
**dadurch gekennzeichnet,**
**dass** der Hohlkörper (12) an seiner dem Augeninneren zugewandten Seite mindestens eine Vorrichtung (42) mit optischer Brechkraft aufweist.

8. Augenimplantat nach einem der vorhergehenden Ansprüche,
**dadurch gekennzeichnet,**
**dass** das Medium (14) flüssig und/oder halb-flüssig und/oder gasförmig ist.
